# FASCICULE DE BREVET EUROPEEN

(11) **EP 1 750 629 B1**
(45) Date de publication et mention de la délivrance du brevet: **09.09.2009**
(21) Numéro de dépôt: 05749156.5
(22) Date de dépôt: 27.05.2005
(51) Int. Cl.: A61F 5/56

(54) **ORTHESE D"AVANCEMENT MANDIBULAIRE**
UNTERKIEFERVORSCHIEBEORTHESE
MANDIBULAR ADVANCER ORTHESIS.

(30) Priorité: 04.06.2004 EP 04102543
(43) Date de publication de la demande: 14.02.2007
(73) Titulaire: Magnin, Georges, 2314 La Sagne (CH)
(72) Inventeur: Magnin, Georges, 2314 La Sagne (CH)
(74) Mandataire: P&TS Patents & Technology Surveys SA
(86) Numéro de dépôt international: PCT/EP2005/052431
(87) Numéro de publication internationale: WO 2005/117776

(56) Documents cités:
- EP-A- 1 312 319
- WO-A-01/80764
- DE-A- 10 065 208
- DE-A1- 3 816 769
- DE-C- 10 216 242
- US-A- 5 829 441
- US-A- 6 109 265
- US-A1- 2002 000 230
- US-B1- 6 170 485
- US-B1- 6 526 982

## Description

### Domaine technique

La présente invention se rapporte au domaine des orthèses buccales, et notamment à une orthèse pour le traitement du ronflement et de l'apnée nocturne.

### Etat de la technique

Le ronflement est une manifestation sonore bien connue dont les causes anatomiques et physiologiques sont liées à une hypotonie musculaire qui apparaît lors de l'endormissement. En effet la mandibule, normalement maintenue par l'action des muscles, tend à reculer pendant le sommeil, entraînant dans son mouvement la langue vers l'arrière de la bouche et provoquant ainsi une diminution du calibre des voies aériennes supérieures. Les turbulences du flux respiratoire qui en résultent, amplifiées par des phénomènes de vibration et de résonance du voile du palais et des parties molles adjacentes sont à l'origine du bruit du ronflement qui peut, dans certains cas, atteindre une intensité extrêmement élevée.

Le ronflement ne se limite pas à une gêne, même sévère, de la qualité du sommeil des familiers et des proches de la personne atteinte. Parfois, et notamment chez les individus présentant une macroglossie ou une cavité buccale de petites dimensions, ou si la laxité des tissus est élevée, l'obstruction du canal de respiration peut être grave ou complète, entraînant de fait des interruptions de la respiration, ou apnées obstructives du sommeil.

Dans ce cas, le ronflement est un aspect d'une véritable pathologie qui se manifeste également par la fragmentation du sommeil entraînant une somnolence diurne et une baisse des capacités de concentration. L'apnée entraîne une hypoxie, une bradycardie suivie d'une tachycardie avec risques hypertensifs. Cette apnée est susceptible de déclencher un arrêt cardio-respiratoire nocturne.

On sait traiter efficacement le ronflement et l'apnée nocturne par port nocturne d'un masque nasal insufflant de l'air sous pression dans le pharynx. Cette solution est néanmoins excessivement contraignante pour le porteur et nécessite des calibrations individuelles pour chaque patient, ainsi que des contrôles d'efficacité polysomnographiques. Pour ces raisons, l'application de ce type de traitement est limitée aux syndromes obstructifs et ne se justifie pas dans le traitement du simple ronflement. A la longue, le traitement est susceptible d'entraîner des manifestations nasales du type de rhinites ou d'escarres cutanées rendant impossible le port du masque.

On sait aussi traiter le ronflement et les apnées du sommeil par une opération de résection du voile du palais, éventuellement complétée par une chirurgie d'avancement maxillaire. Cette solution est toutefois invasive et douloureuse, et l'amélioration qui en dérive est seulement transitoire. De plus, elle peut impliquer des changements de la morphologie buccale pouvant perturber la déglutition. Cette opération très invasive est désormais pratiquement abandonnée.

On a également proposé pour combattre le ronflement, des appareils intra-buccaux s'accrochant aux arcades dentaires supérieure et inférieure, permettant de propulser antérieurement la mandibule; même en l'absence d'une tension musculaire suffisante. La demande de brevet DE198628 décrit un exemple de dispositif monobloc de ce type de type immobilisant complètement la mandibule en position avancée. L'immobilisation de la mandibule est gênante pour le porteur, notamment en ce qu'elle interfère avec la déglutition et peut provoquer des syndromes algo-dysfonctionnels de l'articulation temporo-mandibulaire.

Le brevet EP08459628 décrit un dispositif articulé thermoformable combinant des éléments souples et des éléments semi-rigides, et autorisant une certain liberté de mouvement de la mandibule tout en la propulsant en avant. Ce dispositif se compose de deux coques réalisées en un matériau thermoformable. De cette manière, chaque patient peut adapter les coques à sa propre conformation en les chauffant dans un bain d'eau porté à une température suffisante à ramollir la couche thermoformable, puis en les moulant sur l'arcade correspondante.

La conformation des dents et des arcades dentaires varie toutefois énormément d'un individu à l'autre, soit parce que les arcades peuvent être plus ou moins larges chez chaque individu, soit parce que les dents ne sont pas régulièrement positionnées le long de chaque arcade. Les dispositifs thermoformables connus ne présentent qu'une adaptabilité très limitée et ne peuvent pas s'adapter à tout le spectre de conformations possibles.

Lorsque l'orthèse ne peut pas s'adapter parfaitement à la conformation dentaire individuelle, on constate une réduction d'efficacité, une instabilité du dispositif, des tensions excessives sur la dentition, ainsi qu'une adaptation du système très inconfortable. En plus, suite aux mouvements involontaires de serrage de la mandibule, des efforts mécaniques élevés et concentrés peuvent se produire sur les éléments semi-rigides des coques, pouvant conduire à la rupture du dispositif.

Une autre limitation de ce type de dispositifs est que, si l'orthèse ne s'adapte pas parfaitement à la conformation dentaire, elle peut, pendant l'usage, exercer des forces latérales sur les dents, même de grande intensité. Ces forces latérales peuvent, avec le temps, conduire à un déplacement et à une déstabilisation des dents, et à leur perte définitive.

Il est aussi connu de réaliser des orthèses d'avancement mandibulaire spécialement fabriquées pour s'adapter à la conformation dentaire du porteur. Dans ce cas, chaque orthèse est fabriquée comme une pièce unique d'après les empreintes du maxillaire et de la mandibule du porteur, par des procédés conventionnels dans le domaine de l'orthodontie. On conçoit facilement que le coût de fabrication de ce type d'appareil personnalisé est nettement plus élevé que celui d'une orthèse adaptable fabriquée en série.

Une autre limitation des dispositifs mandibulaires connus, tel que par exemple les dispositifs décrits par EP0845962B, qui autorisent une certaine liberté de mouvement de la mandibule est que cette dernière peut, à la suite de mouvements involontaires dans le sommeil, abandonner la position propulsée en avant. Le dispositif perd alors entièrement son efficacité. Dans ces cas la liberté de rétropulsion va paradoxalement à l'encontre du but recherché, de l'avancement mandibulaire.

Le brevet US5829441 décrit un dispositif d'avancement mandibulaire comprenant des coques flexibles, autorisant la déformation et l'adaptation aux diverses configurations dentaires, lors de la prise de l'empreinte sur l'arcade dentaire du porteur.

La position du porteur pendant le sommeil peut aussi influencer négativement le bon fonctionnement des dispositifs mandibulaires connus. Il est par exemple fréquent que la mandibule abandonne la position propulsée en avant lorsque la tête est positionnée sur le côté.

Il est aussi connu que certaines phases du sommeil sont caractérisées par une activité et une tonicité musculaire très importantes. Une fois ces épisodes de sommeil agité terminés, les dispositifs de l'art antérieur ne sont pas toujours en mesure de reporter automatiquement la mandibule dans la position thérapeutique propulsée.

Une autre limitation des dispositifs personnalisables connus est qu'ils se basent sur une géométrie buccale conventionnelle dans laquelle les dents sont parallèles et rangées sur deus arcades planes. Ces orthèses ne sont pas en mesure de s'adapter à la plupart des conformations dentaires réelles.

### Bref résumé de l'invention

Un but de la présente invention est de proposer une orthèse d'avancement mandibulaire exempte des inconvénients de l'art antérieur.
Un autre but de la présente invention est de proposer un dispositif intra-buccal contre le ronflement pouvant s'adapter à une très grande partie de conformations individuelles sans risques irréversibles pour la dentition de l'utilisateur.
Un autre but de la présente invention est de proposer une orthèse d'avancement mandibulaire ayant une bonne stabilité pour n'importe quel type de conformation dentaire du porteur.

Un autre but de la présente invention est de proposer un dispositif intra-buccal contre le ronflement plus confortable que les dispositifs connus.

Encore un but de la présente invention est de proposer une orthèse d'avancement mandibulaire adaptable à la conformation dentaire du porteur présentant un confort et une efficacité équivalents à ceux d'une orthèse personnalisée et un coût de fabrication réduit.

Ces buts sont atteints par les produits et le procédé qui sont l'objet des revendications indépendantes des catégories correspondantes.

### Brève description des dessins

L'invention sera mieux comprise à la lumière de la description, des revendications et des figures suivantes dans lesquelles notamment :

Les figures 1 et 2 représentent schématiquement une orthèse d'avancement mandibulaire de type connu ;

La figure 3 représente schématiquement un dispositif comprenant des stries de glissement convexes et permettant un passage d'air selon un aspect de la présente invention.

La figure 4 représente schématiquement un dispositif comprenant une armature flexible externe selon un aspect de la présente invention.

La figure 5 représente schématiquement une variante ultérieure d'un dispositif comprenant une armature flexible externe selon un aspect de la présente invention.

La figure 6 représente schématiquement un dispositif comprenant une armature flexible interne selon un aspect de la présente invention.

Les figures 7 et 8 représentent schématiquement deux sections du dispositif de la figure 5.

La figure 9 représente schématiquement une section du dispositif de la figure 5 inséré dans une gouttière de prise d'empreinte.

La figure 10 représente schématiquement un indicateur de température étalonné utilisé dans le procédé de prise d'empreinte selon l'invention.

Les figures 11 et 12 représentent schématiquement un autre mode de réalisation de l'invention comprenant une surface inclinée par rapport au plan de glissement principal.

Les figures 13, 14 et 15 représentent schématiquement un autre mode de réalisation de l'invention.

Les figures 16 et 17 montrent schématiquement un autre mode de réalisation de l'invention comprenant une languette élastique.

La figure 18 représente schématiquement une coupe de l'extrados d'une orthèse selon l'invention selon le plan AA de la figure 12.

La figure 19 représente schématiquement une vue du haut de l'élément inférieur de l'orthèse selon l'invention, selon une variante préférentielle du mode de réalisation de la figure 6.

Les figures 20, 21, 22 et 23 représentent schématiquement et en trois dimensions les éléments supérieurs et inférieurs d'un dispositif selon l'invention, ainsi que les armatures internes flexibles respectives.

### Exemple(s) de modes de réalisation de l'invention

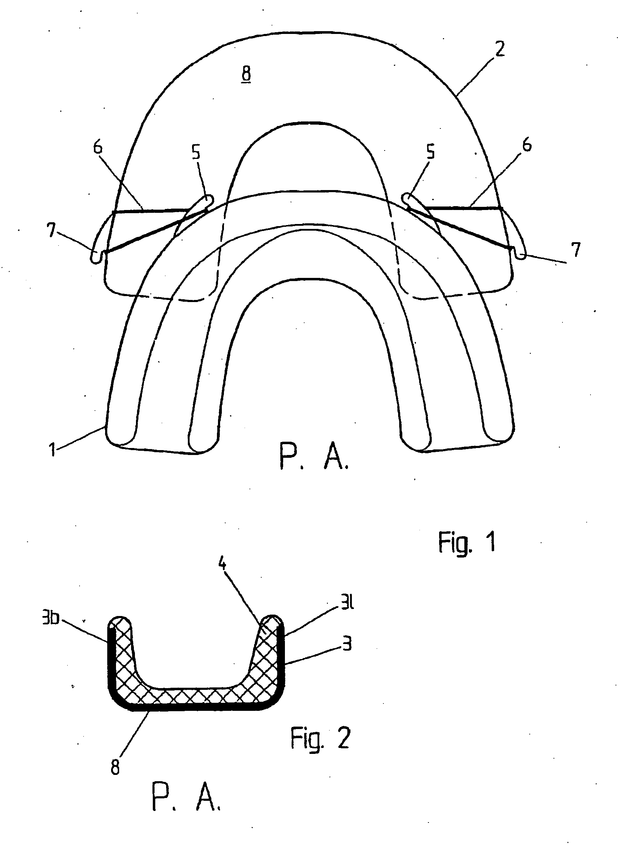

La figure 1 représente un dispositif intra-buccal contre le ronflement de type connu. Il se compose de deux coques 1, 2, destinées à envelopper l'arcade dentaire supérieure, respectivement inférieure. Avec référence à là figure 2, les coques 1 et 2 présentent un extrados essentiellement rigide 3 sur lequel un intrados thermoformable 4 est surmoulé. Les coques sont reliées entre elles par les crochets 5 et 7 et les boucles élastiques 6, pour exercer une force de propulsion sur la mandibule, tout en permettant une certaine liberté de mouvement. L'extrados rigide de ce dispositif est incapable de suivre une dentition irrégulière.

Lors de l'usage, les surfaces planes 8 des deux extrados se trouvent en contact le long du plan d'occlusion. Les mouvements de latéralité et de propulsion/rétropulsion de la mandibule comportent donc un glissement de ces surfaces 8 l'une sur l'autre.

Pour permettre une adaptation personnalisée de cet appareillage chaque patient peut adapter les coques à sa propre conformation en moulant l'intrados thermoformable sur l'arcade correspondante, après l'avoir ramolli par immersion dans un bain d'eau à une température appropriée.

Le dispositif de la figure 1 présente donc une bonne conformabilité pour autant que toutes les dents se positionnent en correspondance de l'intrados souple. Il est toutefois fréquent que les dents ne suivent pas exactement la courbure des coques 1 et 2, et qu'une dent se trouve en correspondance de l'extrados 3 lors de la prise de l'empreinte. En cette situation l'orthèse ne peut pas du tout s'adapter à l'arcade.

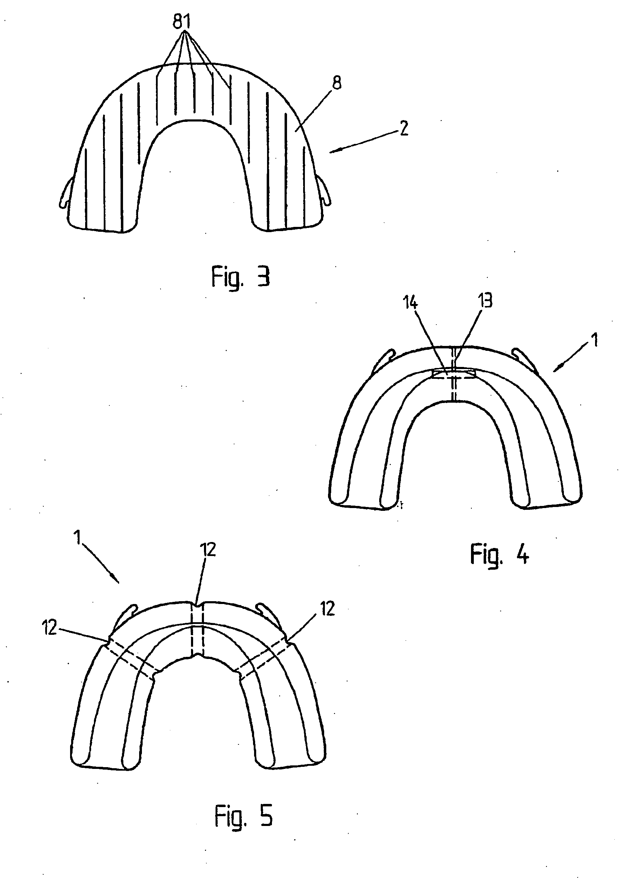

La figure 4 représente une coque d'un dispositif comprenant une armature articulée selon l'invention. Dans ce dispositif, l'extrados comprend au moins un joint flexible de manière à réaliser une armature articulée pour suivre la conformation dentaire du patient, même dans le cas d'une implantation irrégulière des dents.

Dans cette forme d'exécution simple, le dispositif de l'invention se limite au seul point central de flexion, situé en correspondance du plan sagittal. Le joint flexible est obtenu en pratiquant une fente 13 dans l'extrados 3. La flexibilité relativement plus élevée en correspondance de la fente 13 permet l'articulation de l'extrados 3. Un élément flexible métallique ou plastique 14 peut être inséré pour relier les deux moitiés droite et gauche de l'extrados 3. Le système de l'invention peut comprendre une ou plusieurs fentes selon le degré d'adaptabilité recherché.

La figure 5 représente, par souci de simplification, la coque supérieure 1 uniquement. Bien entendu, les caractéristiques propres à ce mode de réalisation de l'invention peuvent aussi être avantageusement incorporées dans la coque inférieure 2.

La fente centrale 13 permet aussi l'alignement correct des deux coques lors de la prise d'empreinte. Optionnellement la même fonction peut être assuré par des marques, de préférence convexes, gravées à cet effet sur les deux coques.

Dans le cadre de la présente invention, les boucles élastiques pourraient être remplacée par d'autres moyens équivalents, par exemple par des ressorts, des pistons, par des languettes flexibles réalisés dans l'une ou dans l'autre coque ou par des aimants.

Avantageusement, l'armature comporte une pluralité de joints flexibles, pour s'adapter à un plus grand spectre de conformations dentaires. La figure 5 représente une coque d'un dispositif selon l'invention muni d'un extrados formant une armature articulée. L'extrados 3 comporte dans ce cas trois joints flexibles 12 réalisés par un amincissement des parois externes. De préférence, l'extrados 3 sera réalisé en un polymère susceptible d'être formé par moulage, comportant une bonne élasticité et pouvant tolérer un grand nombre de cycles de flexion, par exemple en un élastomère thermoplastique.

Les matériaux composants l'extrados et l'intrados sont sélectionnés afin de procurer une bonne adhésion entre ces deux parties. Une bonne compatibilité chimique est donc importante pour parvenir à ce résultat. L'adhésion entre extrados et intrados peut être encore améliorée en prévoyant des nervures ou des cavités dans l'extrados pour offrir une surface d'accrochage lors du surmoulage.

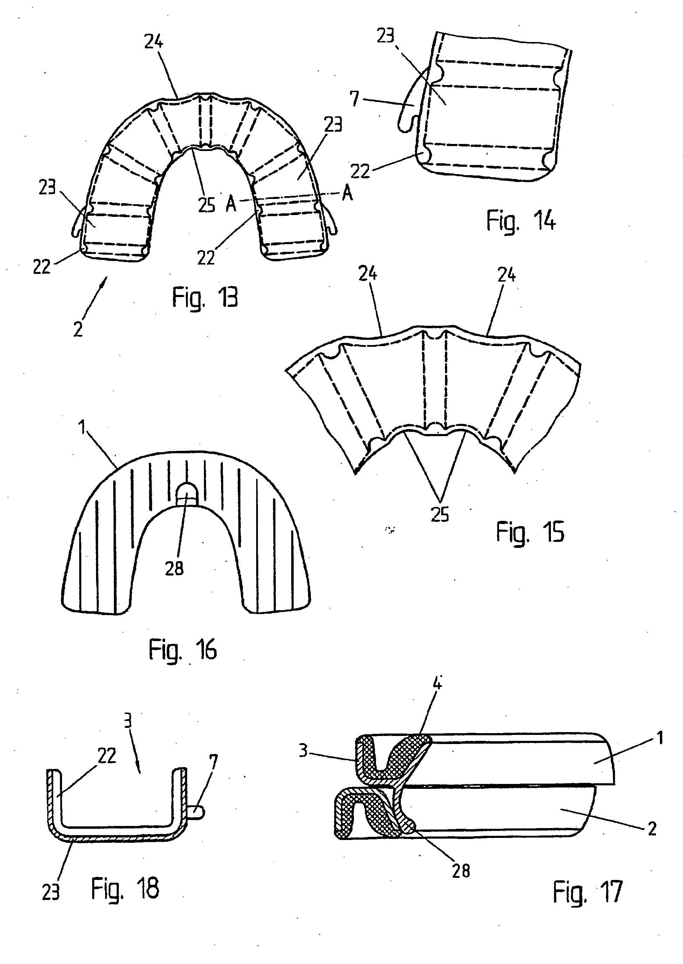

Les figures 13, 14 et 15 montrent l'extrados flexible de la coque inférieure 8 d'une orthèse selon une autre variante de l'invention.

Dans ce mode de réalisation les joints flexibles sont réalisés par amincissement des parois intérieures de l'extrados, ce qui présente l'avantage de conserver une surface extérieure parfaitement lisse, et de ne pas laisser des surfaces extérieures concaves, difficiles à nettoyer. L'extrados comporte selon cette variante une alternance de sections minces 23 et de nervures plus rigides 22. Les sections minces 23 travaillent essentiellement en flexion et en extension, et permettent à l'extrados de suivre la forme générale de l'arcade dentaire. Les nervures 22, en revanche, présentent une rigidité suffisante pour garantir la stabilité latérale de l'orthèse contre les dents, et notamment contre les faces linguales et labiales des dents.

Il est important, pour la stabilité de l'orthèse et pour la santé dentaire, que les flancs 3l et 3b de l'extrados exercent, par leur rigidité, deux force latérales opposées sur les faces linguales et labiales des dents, respectivement. L'orthèse de l'invention, par sa conformabilité, permet le positionnement symétrique des deux flancs 3l et 3b par rapport aux dents, et par conséquent une tenue efficace et sans efforts asymétriques, même en présence d'un positionnement irrégulier des dents.

La conformabilité des coques de l'invention, permet de répartir correctement la traction exercée par les boucles élastiques 6, sur un grand nombre de dents, typiquement 20 ou plus. On évite de cette manière les dégâts collatéraux qui pourraient surgir si la force de propulsion était appliquée à un nombre limité de dents (par ex. déformation du plan d'occlusion ou déplacement des dents).

Avantageusement les zones plus sollicitées en extension et en compression présentent des parois minces courbées 24, 25 pour pouvoir assurer des élongations et des compressions plus importantes, grâce à leur flexibilité. La courbure des parois 24 et 25 pourra être vers l'intérieur (concave) ou vers l'extérieur (convexe), selon la nécessité.

La figure 18 représente schématiquement une coupe de l'extrados 3 selon le plan AA de la figure 13. On peut voir la section mince 23, traversée par le plan de coupe AA, et la nervure 22 en arrière plan.

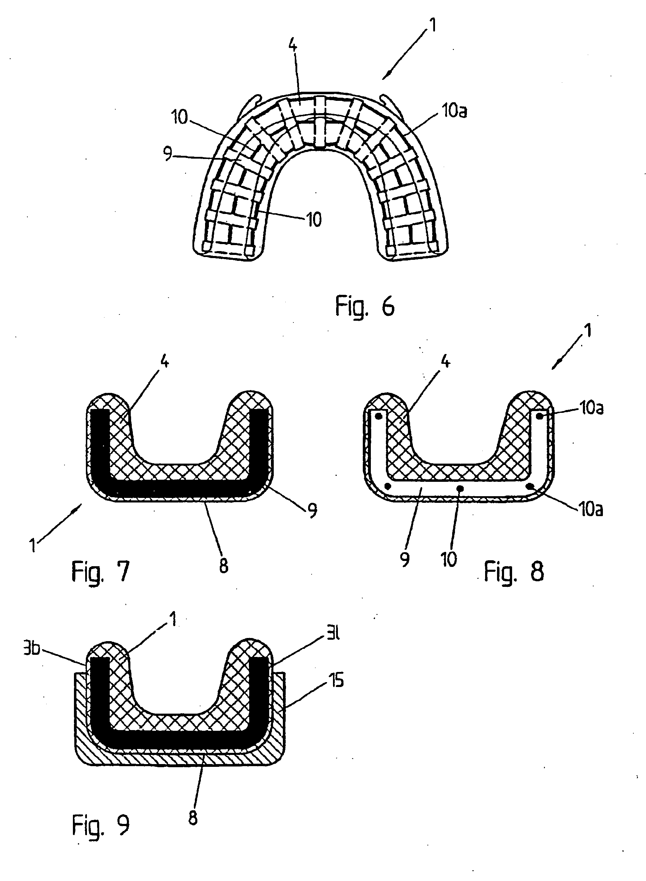

Un autre mode de réalisation de l'invention sera maintenant décrit avec référence à la figure 6. Dans ce mode de réalisation une armature flexible et articulée est insérée à l'intérieur d'une coque 4 en un matériau souple et thermoformable. L'armature flexible se compose d'une succession d'éléments 9 en forme de « U » rigides, ouverts vers l'arcade dentaire, reliés par des éléments flexibles 10 et 10a plus minces. Avantageusement l'armature articulée est réalisée en une seule pièce par moulage d'un matériau approprié comportant une bonne élasticité est déformabilité et pouvant tolérer un très grand nombre de cycles de flexion en section mince.

Les articulations flexibles 10 et 10a permettent la déformation de l'armature en les trois directions de l'espace, horizontalement et verticalement, ainsi que des mouvements de torsion. De cette manière l'orthèse de l'invention peut épouser parfaitement la conformation dentaire en trois dimensions, par exemple de s'adapter à la courbure naturelle de la surface d'occlusion dentaire, ou à un implantation non parallèle des dents. (Les désignations "horizontal" et "vertical" se référent ici à l'orientation conventionnelle de l'orthèse lorsque elle est portée par un porteur en position débout.)

L'orthèse selon cette variante de l'invention présente une surface extérieure entièrement souple, et offre donc un confort supérieur aux dispositifs comportant des surfaces extérieures rigides ou semi-rigides. Les figures 7 et 8 montrent deux coupes de la coque 4 de la figure 6 en correspondance des éléments 9 et 10.

Un autre avantage de cette variante de l'invention est que les risques de décollement entre l'armature interne et la partie thermo-formable sont inexistants. La coque thermoformable 4 est surmoulée autour de l'armature flexible et enrobe complètement celle-ci.

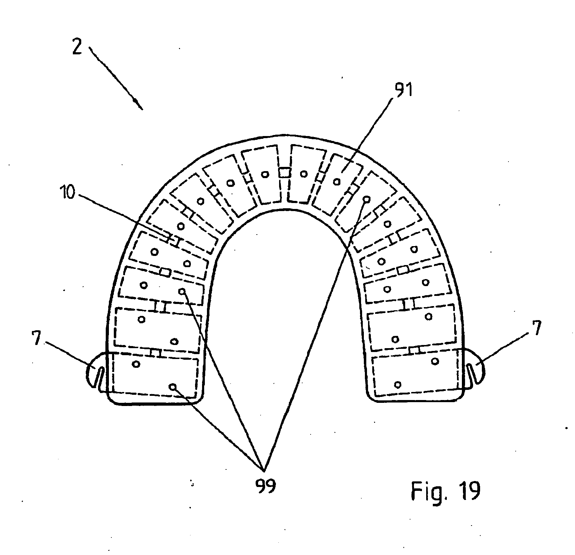

La figure 19 représente une vue du haut de élément inférieur 2 de l'orthèse de l'invention réalisé selon une variante préférentielle du mode de réalisation de l'invention que l'on vient de décrire. En cette variante l'armature articulée flexible, visible en transparence dans la figure, se compose d'un plus grand nombre d'éléments 91 en forme de "U" relativement rigides, ouverts vers l'arcade dentaire, reliés par une arête relativement flexible 10, ou un autre élément flexible qui relie les éléments 91. l'armature articulée est réalisée en une seule pièce par moulage d'un matériau approprié comportant une bonne élasticité et déformabilité un fluage minime et pouvant tolérer un très grand nombre de cycles de flexion en section mince, tel par exemple le polyacétal de qualité médicale. Avantageusement les crochets 7 prévus pour la fixation de l'élastique, sont réalisés intégralement dans l'armature.

Préférablement, les éléments relativement rigides 91 comportent des orifices 99 pour faciliter le passage de la matière thermoformable, lors du surmoulage de la coque 4 autour de l'armature. Cette caractéristique pourra aussi être adoptée dans les autres variantes de l'invention ci décrites, selon les cas.

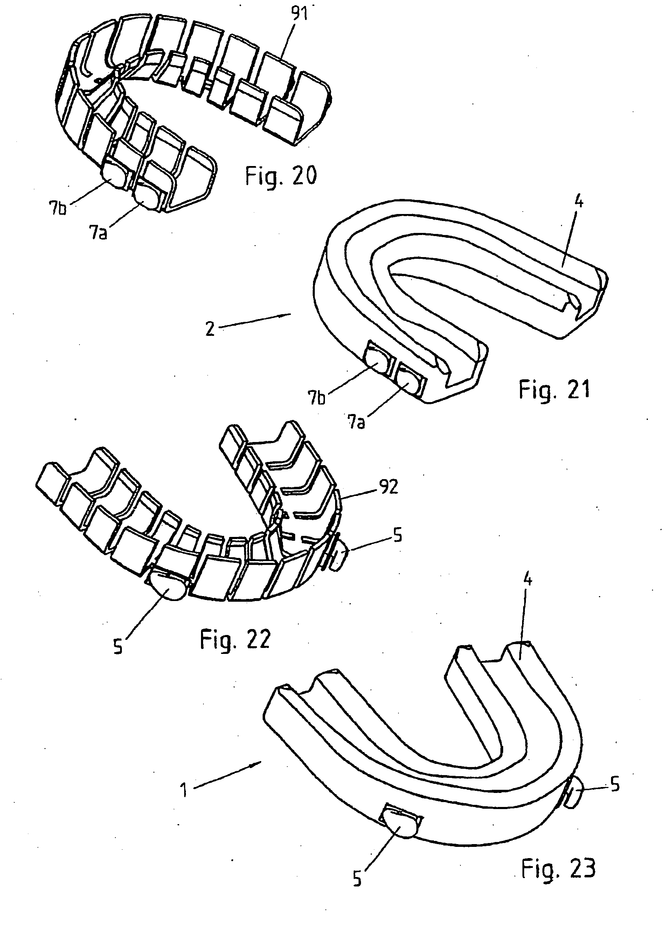

La figure 20 montre en trois dimensions l'armature interne inférieure de la figure 19. Dans cette variante l'armature porte deux paires de crochets supplémentaires 7a, 7b, au lieu qu'une seule paire. Cette disposition permet de moduler de manière simple la force de propulsion exercée par les bandes élastiques (non représentées) simplement en choisissant le crochet d'attache

La figure 21 montre, en trois dimensions, la gouttière inférieure 2, après le surmoulage de la coque thermoformable 4.

La figure 22 représente, de manière correspondante à la figure 20, une armature pour une gouttière supérieure comprenant un certain nombre d'éléments 92 en forme de "U" relativement rigides, ouverts vers l'arcade dentaire, reliées par des arêtes flexibles. La figure 23 montre, la gouttière supérieure 1 après le surmoulage de la matière thermoformable 4.

Cette variante de l'invention présente l'avantage que les gouttières peuvent se déformer, lors de la prise d'empreinte, selon toutes les directions dans l'espace. L'armature de l'invention peut non seulement s'ouvrir et se refermer dans le plan horizontal, pour s'adapter à une mandibule ou à une mâchoire plus ou moins ouverte, mais elle peut aussi se courber verticalement, pour épouser la courbure de la surface d'occlusion, et se tordre suivant la direction d'implantation des dents.

Grâce à cette caractéristique l'orthèse de l'invention peut s'adapter en trois dimensions à toute conformation dentaire du porteur.

La figure 9 illustre un élément d'interface 15 qui intervient lors de la prise d'empreinte du dispositif de l'invention. Le but de ce dispositif est d'empêcher, pendant la prise d'empreinte, un écartement des deux flancs 31 et 3b, qui aurait l'effet de réduire la pression sur les faces linguales et labiales des dents et, en conséquence, la stabilité de l'orthèse. A cette fin, les coques sont insérées, pour la prise d'empreinte seulement, dans deux éléments d'interface en forme de gouttières souples jetables 15 qui empêchent la déformation de la surface 8 et l'écartement des francs 31 et 3b.

Il est également important que les deux coques qui composent l'orthèse présentent deux plans de glissement 8 parfaitement adaptés l'un à l'autre. L'usage des éléments d'interface 15 permet d'empêcher toute déformation desdits plans lors de la prise d'empreinte.

Une gouttière symétrique est prévue pour la coque inférieure. Une fois terminé le moulage des gouttières 1 et 2 sur les arcades, les éléments d'interface 15 peuvent être éliminés, après refroidissement des éléments thermoformables 4.

Ces éléments d'interface 15 peuvent aussi être avantageusement employés dans les autres modes de réalisation présentés, tout en restant dans le cadre de la présente invention.

Optionnellement, le plan de glissement 8 d'au moins une des coques 1 et 2 présente une pluralité de stries convexes parallèles 81, ou d'autres reliefs pour améliorer le glissement comme représenté sur la figure 3.

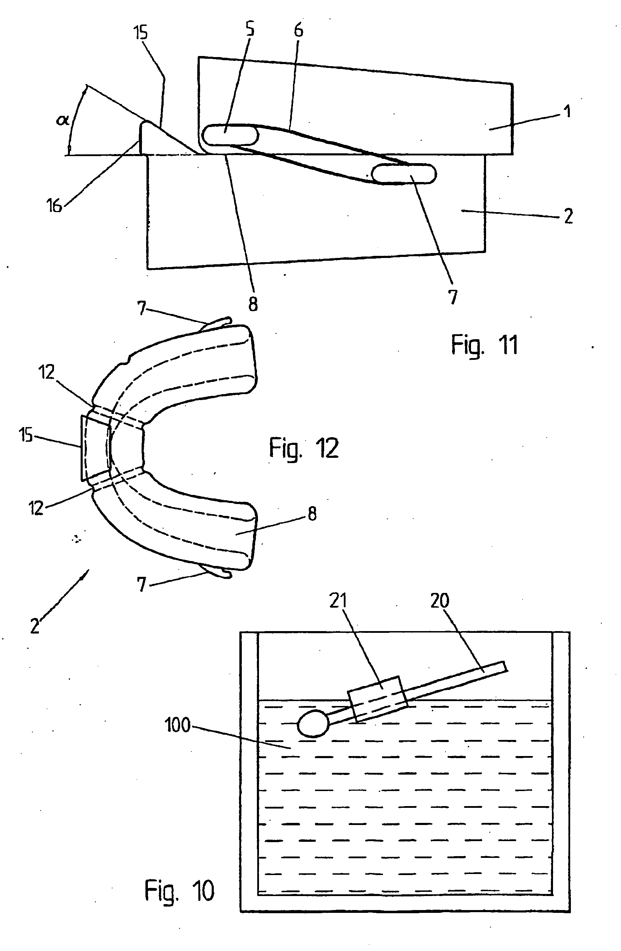

Avec référence aux figures 11 et 12, la coque inférieure 2 présente, à l'extrémité antérieure une protubérance 16 ayant un plan incliné 15, orienté dans le sens d'augmenter la force de propulsion lors d'un mouvement de rétropulsion de la mandibule, aidant ainsi le maintien de la position propulsée. On a vu que, pendant le sommeil, la mandibule se déplace parfois vers l'arrière de façon involontaire. Ces mouvements sont physiologiques pendant le sommeil et la déglutition et il ne serait pas envisageable de bloquer la mandibule dans une position propulsée statique. Cette variante facilite le retour de la mandibule à la position avancée après ces mouvements.

La figure 10 représente un thermomètre pour la prise de température d'un bain d'eau chaude destiné à faciliter la mise en forme des dispositifs de l'invention.

La prise d'empreinte comporte l'immersion préalable des coques dans un bain d'eau froide amené ensuite à une température d'environ 75 °C.

Les thermomètres traditionnels en verre on tendance à se déposer sur le fond du bain 100, et vont donc relever une température bien supérieure à la température prescrite du bain. Par ailleurs les dispositifs trop légers, par exemple ceux utilisant à cristaux liquides sont facilement transportés par les mouvements du liquide bouillonnant, et leur lecture n'est pas facile.

Le thermomètre 20 comporte un flotteur asymétrique 21 déterminant une position d'équilibre hydrostatique inclinée par rapport à la verticale du thermomètre 20. Cette disposition permet une lecture aisée de la température. La position d'équilibre du thermomètre est choisie, de préférence, de manière à ce que le bulbe sensible du thermomètre se trouve, dans l'eau, à la même hauteur des coques flottantes 1 et 2.

Avec références aux figures 16 et 17 un autre mode de réalisation de la présente invention prévoit une languette flexible élastique 28 réalisée intégralement dans la coque supérieure 1 lors du moulage. D'autres positions, par exemple postérieurement, sont toutefois possibles et comprises dans la présente invention.

La languette 28 appuie contre la coque inférieure 2 lorsque l'orthèse est dans sa position d'utilisation normale, et exerce une force de propulsion sur la coque inférieure dans le sens postéro-antérieur, positionnant ainsi la mandibule dans la position propulsée recherchée pour empêcher le ronflement, tout en autorisant une liberté suffisante aux mouvements involontaires du sommeil.

## Revendications

1. Orthèse d'avancement mandibulaire, comprenant une première coque (1) pour envelopper l'arcade dentaire supérieure et une seconde coque (2) pour envelopper l'arcade dentaire inférieure de la cavité buccale, l'orthèse comprenant des moyens de propulsion (6, 28) reliant les dites première et seconde coque (1, 2), propres à exercer une force de propulsion sur la dite seconde coque dans le sens postéro-antérieur, au moins une desdites première et seconde coques (1, 2) comprenant une armature articulée, autorisant une déformation selon trois dimensions, pour s'adapter à la conformation dentaire du porteur,
**caractérisée en ce que**
la dite armature articulée est réalisée par une seule pièce injectée comportant des éléments relativement rigides (9), pour exercer une force de maintien contre les faces labiale et linguale des dents, et des éléments souples (10), pour permettre l'articulation et/ou la déformation de la dite armature.

2. Orthèse selon l'une des revendications précédentes, comprenant des éléments en matière plastique thermoformable (4) dans les dites première et seconde coque (1, 2) pour s'adapter à la conformation dentaire du porteur.

3. Orthèse selon l'une des revendications précédentes, dans laquelle lesdits moyens de propulsion comprennent des boucles élastiques (6).

4. Orthèse selon la revendication 2, dans laquelle la dite armature est à l'intérieur desdits éléments en matière plastique thermoformable (4).

5. Orthèse selon l'une des revendications précédentes, dans laquelle la dite armature comprend une pluralité d'éléments de préhension (9, 91, 92) ouverts vers l'arcade dentaire et aptes à exercer une action de maintien sur les faces dentales et linguales des dents, lesdits éléments de préhension étant reliés par des articulations flexibles (10).

6. Orthèse selon l'une des revendications précédentes, dans laquelle la dite première coque (1) et la dite seconde coque (2) comprennent des indices visuels pour faciliter l'alignement relatif desdites première et seconde coques.

7. Procédé d'adaptation d'une orthèse d'avancement mandibulaire selon l'une des revendications précédentes comprenant les étapes de :
- chauffer la dite orthèse par immersion dans un bain d'eau amené à une température prédéterminée ;
- insérer la première coque et la seconde coque de la dite orthèse dans un premier, respectivement second, élément d'interface amovible (15);
- positionner la dite orthèse dans la bouche du porteur ;
- exercer une pression pour mouler lesdits éléments thermoformables sur les arcades du porteur ;
- ôter lesdits premier et second élément d'interface amovible (15).

8. Procédé selon la revendication précédente, **caractérisé en ce que** la température dudit bain d'eau (100) est déterminée par lecture d'un indicateur de température muni d'un flotteur (21), la position d'équilibre hydrostatique dudit indicateur de température étant inclinée par rapport à la verticale.

## Claims

1. Mandibular advancement splint, having a first tray (1) for enveloping the upper dental arch and a second tray (2) for enveloping the lower dental arch of the mouth cavity, the splint including advancement means (6, 28) connecting said first and second trays (1, 2), capable of exerting a advancement force onto said second tray in the postero-anterior direction, at least one of said first and second trays (1, 2) including an articulated frame, allowing deformation along three dimensions, to adapt to the wearer's dental conformation,
**characterized in that**
said articulated frame is made of a single injected piece having relatively rigid elements (9) to exert a holding force against the labial and lingual sides of the teeth, and flexible elements (10) to allow said frame to articulate and/or deform.

2. Mandibular advancement splint according to the preceding claim, having elements of thermoformable plastic material (4) in said first and second trays (1, 2) to adapt to the wearer's dental conformation.

3. Mandibular advancement splint according to one of the preceding claims, wherein said advancement means include elastic loops (6).

4. Mandibular advancement splint according to claim 2, wherein said frame is inside said elements of thermoformable plastic material (4).

5. Mandibular advancement splint according to one of the preceding claims, wherein said frame includes a plurality of prehension elements (9, 91, 92) open towards the dental arch and capable of exerting a holding action on the dental and lingual sides of the teeth, said prehension elements being connected by flexible articulations (10).

6. Mandibular advancement splint according to one of the preceding claims, wherein said first tray (1) and said second tray (2) have visual indicia to make easier the relative alignment of said first and second trays.

7. Method for adapting a mandibular advancement splint according to one of the preceding claims, including the steps of:
- heating said splint by immersing it in a water bath brought to a predetermined temperature;
- inserting the first tray and the second tray of said splint in a first respectively second removable interface element (15);
- positioning said splint in the wearer's mouth;
- exerting a pressure for splint said thermoformable elements onto the wearer's arches;
- removing said first and second removable interface element (15).

8. Method according to the preceding claim, **characterized in that** the temperature of said water bath (100) is determined by reading a temperature indicator provided with a floater (21), the hydrostatic equilibrium position of said temperature indicator being inclined relatively to the vertical axis.

## Patentansprüche

1. Unterkiefervorschiebeorthese, mit einer ersten Schale (1), um den oberen Zahnbogen (Arcus dentalis maxillaris) zu umhüllen, und einer zweiten Schale (2), um den unteren Zahnbogen (Arcus dentalis mandibularis) der Mundhöhle zu umhüllen, wobei die Orthese Verschiebungsmittel (6, 28) umfasst, welche die besagte erste und zweite Schale (1, 2) verbinden und fähig sind, eine Verschiebungskraft auf die besagte zweite Schale in die posteroanteriore Richtung auszuüben, wobei mindestens eine der besagten ersten und zweiten Schalen (1, 2) einen artikulierten Rahmen umfasst, welche eine Verformung entlang drei Dimensionen ermöglicht, um sich an die Zahnanordnung des Trägers anzupassen,
**dadurch gekennzeichnet, dass**
der artikulierte Rahmen aus einem einzigen injizierten Stück ausgeführt ist, mit relativ rigiden Elementen (9), um eine Haltekraft gegen die labiale und linguale Seite der Zähne auszuüben, und mit flexiblen Elementen (10), um die Artikulation und/oder Verformung des besagten Rahmens zu erlauben.

2. Orthese gemäss dem vorhergehenden Anspruch, mit Elementen aus hitzeverformbarem Kunststoff (4) in den besagten ersten und zweiten Schalen (1, 2), um sich an die Zahnordnung des Trägers anzupassen.

3. Orthese gemäss einem der vorhergehenden Ansprüche, worin die besagten Verschiebungsmittel elastische Schleifen (6) umfassen.

4. Orthese gemäss einem der vorhergehenden Ansprüche, worin der besagte Rahmen innerhalb der besagten Elementen aus hitzeverformbarem Kunststoff (4) ist.

5. Orthese gemäss einem der vorhergehenden Ansprüche, worin der besagte Rahmen eine Vielzahl von Greiferelementen (9, 91, 92) umfasst, welche gegen den Zahnbogen offen sind und fähig sind, eine Halteeinwirkung auf die dentalen und lingualen Seiten der Zähne auszuüben, wobei die besagten Greifelemente durch flexible Gelenke (10) verbunden sind.

6. Orthese gemäss einem der vorhergehenden Ansprüche, worin die besagte erste Schale (1) und die besagte zweite Schale (2) visuelle Zeichen umfassen, um die relative Ausrichtung der besagten ersten und zweiten Schalen zu erleichtern.

7. Verfahren zur Anpassung einer Unterkiefervorschiebeorthese gemäss einem der vorhergehenden Ansprüche, mit den folgenden Schritten:
- die besagte Orthese durch Eintauchen in einem zu einer vorbestimmten Temperatur gebrachten Wasserbad zu heizen;
- die erste Schale und die zweite Schale der besagten Orthese in ein erstes bzw. zweites abnehmbares Schnittstellenelement (15) einzufügen;
- die besagte Orthese im Munde des Trägers zu positionieren;
- einen Druck auszuüben, um die besagten hitzeverformbaren Elemente auf den Zahnbögen des Trägers zu formen;
- die besagten erste und zweite abnehmbaren Schnittstellenelemente (15) abzunehmen.

8. Verfahren gemäss dem vorhergehenden Anspruch, **dadurch gekennzeichnet, dass** die Temperatur des besagten Wasserbades (100) durch Lesen eines Temperaturanzeigers mit einem Schwimmer (21) bestimmt wird, wobei die hydrostatische Gleichgewichtsposition des besagten Temperaturanzeigers in Bezug auf die Senkrechte geneigt ist.
